# FASCICULE DE BREVET EUROPEEN

(11) **EP 1 007 097 B1**
(45) Date de publication et mention de la délivrance du brevet: **17.10.2001**
(21) Numéro de dépôt: 98906986.9
(22) Date de dépôt: 06.02.1998
(51) Int. Cl.: A61K 48/00, A61K 9/127

(54) **FORMULATION DE PARTICULES AGENT(S) DE TRANSFECTION CATIONIQUE(S)/ACIDES NUCLEIQUES STABILISEES**
FORMULIERUNG VON STABILISIERTER KATIONISCHEN TRANSFEKTIONSMITTEL/NUKLEINSÄUREN PARTIKELN
FORMULATION OF STABILISED CATIONIC TRANSFECTION AGENT(S)/NUCLEIC ACID PARTICLES

(30) Priorité: 10.02.1997 FR 9701467
(43) Date de publication de la demande: 14.06.2000
(73) Titulaire: Aventis Pharma S.A., 92160 Antony (FR)
(72) Inventeur: CROUZET, Joel, F-92330 Sceaux (FR); PITARD, Bruno, F-92330 Sceaux (FR)
(86) Numéro de dépôt international: FR9800222
(87) Numéro de publication internationale: WO9834648

(56) Documents cités:
- WO-A-96/04932
- WO-A-96/30051
- WO-A-97/11682
- LIU ET AL: "NEW CATIONIC LIPID FORMULATIONS FOR GENE TRANSFER" PHARMACEUTICAL RESEARCH, vol. 13, no. 12, 1996, pages 1856-1860, XP002045397
- HARA ET AL: "EMULSION FORMULATIONS AS A VECTOR FOR GENE DELIVERY IN VITRO AND IN VIVO" ADVANCED DRUG DELIVERY REVIEWS, vol. 24, no. 2 3, 17 mars 1997, pages 265-271, XP002067903

## Description

La présente invention se rapporte à des associations agent(s) de transfection cationique(s)/ADN dont les particules sont stabilisées en taille à l'aide d'un agent de surface non ionique et à leurs utilisations en thérapie génique.

De nombreuses maladies génétiques sont associées à un défaut d'expression et/ou une expression anormale, c'est à dire déficiente ou excessive, d'un ou plusieurs acides nucléiques. La thérapie génique a pour principal objectif de corriger ce type d'anomalies génétiques par le biais de l'expression cellulaire in vivo ou in vitro de gènes clonés.

Aujourd'hui, plusieurs méthodes sont proposées pour la délivrance intracellulaire de ce type d'information génétique. L'une d'entre elles, en particulier, repose sur l'emploi de vecteurs chimiques ou biochimiques. Les vecteurs synthétiques ont deux fonctions principales, complexer l'ADN à transfecter et promouvoir sa fixation cellulaire ainsi que son passage à travers la membrane plasmique et, le cas échéant, à travers les deux membranes nucléaires. Parmi les vecteurs synthétiques développés, les polymères cationiques de type polylysine et DEAE dextran ou encore les lipofectants sont les plus avantageux.

Un progrès important a été accompli dans ce mode de transfection avec le développement d'une technologie basée sur l'emploi d'agents de transfection cationiques de type lipofectants et plus précisément de lipides cationiques. Il a ainsi été mis en évidence qu'un lipide cationique chargé positivement, le chlorure de N-[1-(2,3-dioleyloxy)propyl]-N,N,N-triméthylammonium (DOTMA), interférait, sous la forme de liposomes ou de petites vésicules, spontanément avec de l'ADN, qui est chargé négativement, pour former des complexes lipides-ADN, capables de fusionner avec les membranes cellulaires, et permettait ainsi la délivrance intracellulaire de l'ADN.

Depuis le DOTMA, d'autres lipides cationiques ont été développés sur ce modèle de structure : groupe lipophile associé à un groupement amino via un bras dit "spacer". Parmi ceux-ci, on peut plus particulièrement citer ceux comprenant à titre de groupement lipophile deux acides gras ou un dérivé du cholestérol, et comportant, en outre, le cas échéant, à titre de groupement amino, un groupement d'ammonium quaternaire. Les DOTAP, DOBT ou le ChOTB peuvent notamment être cités à titre représentatifs de cette catégorie de lipides cationiques. D'autres composés, comme les DOSC et ChOSC, se caractérisent par la présence d'un groupement choline à la place du groupement d'ammonium quaternaire.

Une autre catégorie de lipofectants, les lipopolyamines, a également été décrite. De manière générale, il s'agit d'une molécule amphiphile comprenant au moins une région hydrophile polyamine associée par une région dite spacer à une région lipophile. La région polyamine des lipopolyamines, chargée cationiquement, est capable de s'associer de manière réversible avec l'acide nucléique, chargé négativement. Cette interaction compacte fortement l'acide nucléique. La région lipophile rend cette interaction ionique insensible au milieu externe, en recouvrant la particule nucléolipidique formée d'une pellicule lipidique. Dans ce type de composés, le groupement cationique peut être représenté par le radical L-5carboxyspermine qui contient quatre groupements ammonium, deux primaires et deux secondaires. Les composés DOGS et DPPES en font notamment partie. Ces lipopolyamines sont tout particulièrement efficaces pour la transfection de cellules endocrines primaires. A titre représentatif de cette dernière famille de composés on peut plus particulièrement faire état des lipopolyamines décrites notamment dans les demandes de brevet WO 96/17823 et WO 97/18185

Toutefois, l'efficacité de ces vecteurs synthétiques reste à améliorer notamment en terme de complexation avec l'acide nucléique et plus précisément sur le plan de la stabilité des particules de ces complexes nucléolipidiques. En effet, avec les formulations classiques acides nucléiques/agent de transfection cationique, on assiste fréquemment et rapidement à un phénomène d'aggrégation des particules de complexes. De tels agrégats possèdent évidemment une taille difficilement compatible avec une transfection thérapeutique. Une des solutions proposées jusqu'ci pour prévenir ce type de phénomène de précipitation, consiste à introduire dans la formulation, l'agent de transfection cationique, comme par exemple le lipofectant, en quantité excessive c'est à dire dans un rapport de charge lipofectant/acides nucléiques de l'ordre de 10 voire plus. Outre le fait qu'une telle solution n'est pas toujours efficace, elle n'est pas totalement satisfaisante sur le plan de l'innocuité. Les agent de transfection cationiques comme les lipofectants et polymères cationiques sont en soit des composés qui, en quantités importantes, risquent de présenter une toxicité relative pour les cellules les incorporant.

Il serait donc particulièrement intéressant dé disposer sur le plan thérapeutique de formulations agents de transfection cationiques/acides nucléiques possédant des rapports de charges réduits et néammoins stabilisées dans le temps sous la forme de particules non aggrégées. Or, comme cela est énoncé précédemment, les zones de concentrations acides nucléiques/lipofectants répondant à de tels rapports de charges sont généralement associées à un état physique instable. On assiste rapidement à un phénomène d'aggrégation des particules nucléolipidiques. Qui plus est, on sait également que la présence d'un sel de type NaCl, classiquement mis en oeuvre dans les formulations agents de transfection cationiques/acides nucléiques, peut induire à certaines concentrations, la précipitation des particules nucléolipidiques. Ainsi, la gamme de concentration en agent de transfection pour laquelle on observe la précipitation sera d'autant plus grande que la concentration en sel sera élevée.

La présente invention a précisément pour objectif de valoriser ces zones de concentrations agent de transfection cationique/acides nucléiques, intéressantes sur le plan de l'innocuité pour leurs quantités réduites en vecteur et également sur le plan interférence avec d'autres protéines compte tenu de leur charge réduite. En effet, lorsque les complexes sont relativement peu chargés, le risque qu'ils interfèrent avec les protéines du sérum in vivo est significativement réduit. Ceci est bien entendu particulièrement avantageux pour le transfert d'acides nucléiques in vivo (Remy, J.S. Proc. Natl. Acad. Sci. USA 1995; 92, 1744-1748). De nombreux articles font état de ce type d'interactions entre les protéines du sérum et des liposomes (Senior, J.H. et al. Biochim. Biophys. Acta 1991, 1070, 173-179; Hernandez-Caselles, T et al. Molecular and cellular Biochemistry 1993, 120, 119-126; Oku, N. et al. Biochim. Biophys. Acta 1996, 1280, 149-154). Des données ont également été publiées sur l'activation du système du complément par des complexes à base d'ADN utilisés pour la thérapie génique. Le degré d'activation du complément dépend du rapport cation/ADN (ou rapport de charge). Ce dernier phénomène est surtout vrai pour les polycations comme les polylysines, les lipospermines (par exemple le DOGS). L'activation du complément est moins sensible au rapport de charge pour les ammoniums quaternaires comme le DOTAP, le DC-Chol, ou le DOTMA (Plank, C. et al. Human Gene Therapy 1996, 7, 1437-1446).

En conséquence, il serait particulièrement intéressant de disposer sur le plan thérapeutique de formulations en complexes agents de transfection cationiques/acides nucléiques à concentration élevée en acides nucléiques et/ou à des rapport de charge réduits voire proches de la neutralité et qui soient en outre stabilisées sous une forme fluide de type colloïdale c'est à dire non agrégées, deux spécificités apparemment naturellement inconciliables.

De manière inattendue, la demanderesse a mis en évidence que l'adjonction d'un agent de surface non ionique à des particules de complexes agent(s) de transfection cationique(s)/acides nucléiques, naturellement instables c'est à dire susceptibles, d'évoluer rapidement vers la formation d'aggrégats, permettait de s'opposer efficacement à ce phénomène et donc de stabiliser les particules de complexes à une taille de particule inférieure à ou de l'ordre de 160 nm.

La présente invention a pour premier objet une composition utile en thérapie génique comprenant des particules de complexes agent(s) de transfection cationique(s)/acides nucléiques caractérisée en qu'elle incorpore en outre au moins un agent de surface non ionique en quantité suffisante pour stabiliser la tailles desdites particules à une dimension inférieure ou égale à 160 nm.

Au sens de l'invention, on entend désigner par des particules stabilisées, des particules dont la taille n'est pas susceptible d'évoluer au cours du temps, lorsque notamment ces particules sont maintenues en dispersion dans une solution. Contrairement aux formulations classiques, c'est à dire exempt d'agent de surface non ionique, les compositions revendiquées peuvent être conservées de manière plus prolongée dans le temps sans que puisse être notée une quelconque modification, de type aggrégation notamment, au niveau de cette dispersion. La taille des particules ainsi stabilisées évolue généralement entre 50 et 160 nm, de préférence entre 75 et 150 nm.

En l'absence de l'agent de surface non ionique revendiqué, les particules de complexes présentes dans la composition revendiquée, conduisent spontanément à des aggrégats particulaires de taille supérieure à 160 nm.

Par ailleurs, la demanderesse a démontré que les compositions selon l'invention possède une structure lamellaire ayant alternativement des bicouches de lipides et des couches d'ADN intercalées.

Les agents de surface non ioniques, conformes à la présente invention, possèdent de préférence au moins un segment hydrophobe et au moins un segment hydrophile. La partie hydrophobe peut être aussi bien une chaîne aliphatique, un polyoxyalkylène, un polyester d'alkylidène, un polyéthylène glycol à tête polyéther benzylique dendritique, ou le cholestérol. Quant à la partie hydrophile, il peut s'agir d'un polyoxyalkylène, d'un alcool polyvinyle, d'un polyvinylpyrrolidone, ou d'un saccharide.

De manière préférée, l'agent de surface mis en oeuvre dans le cadre de la présente invention est ou dérive d'un polyol non ionique et plus particulièrement d'un polyoxyalkylène avec des groupements alkylène de longueurs et/ou de conformations différentes ou non, au sein du polymère.

Plus préférentiellement, il répond à la formule générale suivante :

HO(CH₂CH₂O)ₐ(CH(CH₃)CH₂O)_{b}(CH₂CH₂O)_{c}H

avec a, b et c représentant indépendamment l'un de l'autre des nombres entiers pouvant varier entre 20 et 100 inclusivement.

L'agent non ionique est de préférence présent dans les compositions selon l'invention à une concentration comprise entre 0,01% à 10% poids/volume de ladite composition et plus préférentiellement entre 0,02% et 5% poids/volume.

La présence d'un tel agent de surface non ionique en association avec les complexes est avantageuse à plusieurs titres:

Selon l'invention, les agent(s) de transfection cationique(s) et acides nucléiques sont présents dans la composition dans un rapport de charge qui naturellement est propice au développement d'un phénomène d'aggrégation. Ceci sous-entend que les charges positives portées par le ou les agent(s) de transfection cationique(s) ne sont qu'en léger excès comparativement aux charges négatives portées par l'acide nucléique complexé, voire au mieux les compensent totalement. Un tel rapport de charge est particulièrement avantageux sur le plan in vivo car moins préjudiciable en terme d'interaction avec le sérum ou autres protéines comme par exemple l'albumine et plus intéressant sur le plan innocuité. A titre illustratif, ce rapport de charge évolue de préférence entre 1 et 6 et plus préférentiellement, il est inférieur à 4.

De plus, cet agent de surface est tout à fait compatible avec une administration in vivo. Dans le cas des formulations selon l'invention il n'est en effet pas nécessaire de procéder à une élimination de cet agent de surface non-ionique, préalablement à leur injection dans les cellules à traitées.

Enfin, les compositions selon l'invention peuvent être avantageusement stockées. La présence de l'agent de surface non ionique s'oppose efficacement à tout phénomène de précipitation au sein de ladite composition.

A titre d'agent de surface préféré selon l'invention on citera tout particulièrement le composé de formule générale :

OH(CH₂CH₂O)ₐ(CH(CH₃)CH₂O)_{b}(CH₂CH₂O)_{c}H

avec a égal à 75, b à 30 et c à 75.

D'autres agents de surface non-ioniques préférés sont les polyéthylène glycol à tête polyéther benzylique dendritique, les polyoxyéthylène alcool, ou le polyoxyéthylène nonylphényléther.

Par agent de transfection cationique on désigne de préférence selon l'invention les polymères cationiques et les lipofectants.

En ce qui concerne plus particulièrement les lipofectants, on entend couvrir au sens de l'invention, sous cette dénomination, tout composé ou mélange à caractère lipidique et chargé positivement, déjà proposé à titre d'agent actif à l'égard de la transfection cellulaire d'acides nucléiques.

De manière générale, il s'agit de molécules amphiphiles comprenant au moins une région lipophile associée ou non à une région hydrophile.

A titre représentatif de la première famille de composés, on peut notamment proposer des mélanges lipidiques susceptibles de former des liposomes cationiques. Ces formulations peuvent ainsi contenir du POPC, phosphatidylserine, phosphatidylcholine, cholestérol, lipofectamine ou du maléimidophénylbutyrylphosphatidyléthanolamine associé à un lipide cationique tel que défini ci-après.

Selon un mode particulier de l'invention, l'agent lipofectant mis en oeuvre possède une région cationique.

A titre illustratif de ce type de lipides cationiques construits sur le modèle de structure : groupe lipophile associé à un groupement amino via un bras dit "spacer", on peut plus particulièrement citer le DOTMA et également ceux comprenant à titre de groupement lipophile deux acides gras ou un dérivé du cholestérol, et comportant, en outre, le cas échéant, à titre de groupement amino, un groupement ammonium quaternaire. Les DOTAP, DOBT ou le ChOTB peuvent notamment être cités à titre représentatifs de cette catégorie de lipides cationiques. D'autres composés, comme les DOSC et ChOSC, se caractérisent par la présence d'un groupement choline à la place du groupement ammonium quaternaire.

Avantageusement, les lipofectants convenant à l'invention peuvent également être choisis parmi des lipopolyamines dont la région polyamine répond à la formule générale :

H₂N-(-(CH)ₘ-NH-)ₙ-H

dans laquelle m est un nombre entier supérieur ou égal à 2 et n est un nombre entier supérieur ou égal à 1, m pouvant varier entre les différents groupes de carbone compris entre 2 amines, cette région polyamine étant associée de manière covalente à une région lipophile de type chaîne hydrocarbonée, saturée ou non, du cholestérol, ou un lipide naturel ou synthétique capable de former des phases lamellaires ou héxagonales. Cette région polyamine est plus préférentiellement représentée par la spermine ou un de ses analogues ayant conservé ses propriétés de liaison à l'acide nucléique.

La demande de brevet EP 394 111 décrit des lipopolyamines de cette famille susceptibles d'être mises en oeuvre dans le cadre de la présente invention. A titre représentatif de ces lipopolyamines on peut plus particulièrement citer la dioctadécylamidoglycyl spermine (DOGS) et la 5-carboxyspermylamide de la palmitoylphosphatidylethanolamine (DPPES).

Les lipopolyamines décrites dans la demande de brevet WO 96/17823 peuvent également être utilisées avantageusement selon l'invention. Elles sont représentées par la formule générale: 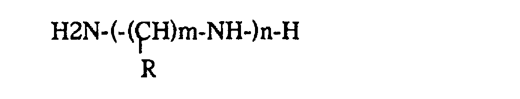 dans laquelle R représente 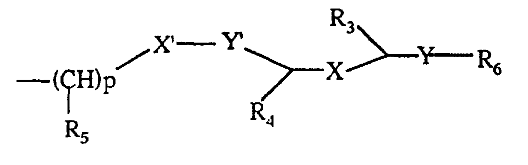 où X et X' représentent, indépendamment l'un de l'autre, un atome d'oxygène, un groupement méthylène -(CH₂)_{q}- avec q égal à 0, 1, 2 ou 3, ou un groupement amino -NH- ou -NR'-, avec R' représentant un groupement alkyle en C₁ à C₄, Y et Y' représentent indépendamment l'un de l'autre un groupement méthylène, un groupement carbonyle ou un groupement C=S, R₃, R₄ et R₅ représentent indépendamment l'un de l'autre un atome d'hydrogène ou un radical alkyle, substitué ou non, en C₁à C₄, avec p pouvant varier entre 0 et 5, R₆ représente un dérivé du cholestérol ou un groupement dialkylamino -NR₁R₂ avec R₁ et R₂ représentant indépendamment l'un de l'autre un radical aliphatique, saturé ou non, linéaire ou ramifié en C₁₂ à C₂₂.

A titre représentatif de ces lipopolyamines on peut tout particulièrement citer le (Dioctadécylcarbamoylméthoxy)-acétate de 2-5-bis-(3-amino-propylamino)-pentyle et le (Dioctadécylcarbamoylméthoxy)-acétate de 1,3-bis-(3-amino-propylamino)-2-propyle.

Enfin, plus récemment, de nouvelles lipopolyamines, valorisables également dans le cadre de la présente invention, ont été décrites dans la demande de brevet WO 97/18185. Il s'agit de composés de formule générale comme suit :

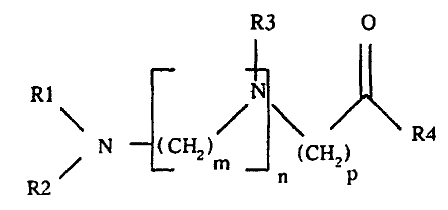

Dans laquelle R₁, R₂ et R₃ représentent indépendamment l'un de l'autre un atome d'hydrogène ou un groupement -(CH₂)_{q}-NRR' avec q pouvant varier entre 1, 2, 3, 4, 5 et 6 ceci de manière indépendante entre les différents groupements R₁, R₂ et R₃ et R et R' représentant indépendamment l'un de l'autre un atome d'hydrogène ou un groupement -(CH₂)_{q},-NH₂, q' pouvant varier entre 1, 2, 3, 4, 5 et 6 ceci de manière indépendante entre les différents groupements R et R', m, n et p représentent, indépendamment l'un de l'autre, un nombre entier pouvant varier entre 0 et 6 avec lorsque n est supérieur à 1, m pouvant prendre des valeurs différentes et R₃ des significations différentes au sein de la formule générale et R₄ représente un groupement de formule générale : 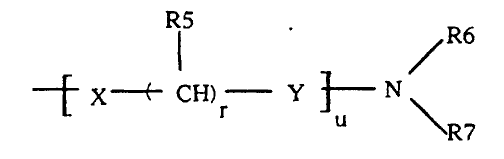 dans laquelle R₆ et R₇ représentent indépendamment l'un de l'autre un atome d'hydrogène ou un radical aliphatique, saturé ou non, en C₁₀ à C₂₂ avec au moins l'un des deux groupements différent de l'hydrogène, u est un nombre entier choisi entre 0 et 10 avec lorsque u est un entier supérieur à 1 R₅, X, Y et r pouvant avoir des significations différentes au sein des différents motifs [X-(CHR₅)ᵣ-Y], X représente un atome d'oxygène, de soufre ou un groupement amine monoalkylé ou non, Y représente un groupement carbonyle ou un groupement méthylène, R₅ représente un atome d'hydrogène ou une chaîne latérale d'acide aminé naturel, le cas échéant substituée et r représente un entier variant entre 1 et 10 avec lorsque r est égal à 1, R₅ représentant une chaîne latérale d'acide aminé naturel substitué ou non et lorsque r est supérieur à 1, R₅ représentant un atome d'hydrogène.

A titre représentatif de ces lipopolyamines on peut plus particulièrement mentionner celles qui suivent :

{H₂N(CH₂)₃}₂N(CH₂)₄N{(CH₂)₃NH₂}(CH₂)₃NHCH₂COGlyN[(CH₂)₁₇CH₃]₂

H₂N(CH₂)₃NH(CH₂)₄NH(CH₂)₃NHCH₂COGlyN[(CH₂)₁₇CH₃]₂

H₂N(CH₂)₃NH(CH₂)₄NH(CH₂)₃NHCH₂COArgN[(CH₂)₁₇CH₃]₂

De manière particulièrement avantageuse, on peut utiliser dans le cadre de l'invention la lipofectamine, la dioctadécylamidoglycyl spermine (DOGS) la 5-carboxyspermylamide de la palmitoylphosphatidylethanolamine (DPPES), le (dioctadécylcarbamoylméthoxy)-acétate de 2-5-bis-(3-amino-propylamino)-pentyle, le (dioctadécylcarbamoylméthoxy)acétate de 1,3-bis-(3-aminopropylamino)-2-propyle, {H₂N(CH₂)₃}₂N(CH₂)₄N{(CH₂)₃NH₂}(CH₂)₃NHCH₂COGlyN[(CH₂)₁₇CH₃]₂. le H₂N(CH₂)₃NH(CH₂)₄NH(CH₂)₃NHCH₂COGlyN[(CH₂)₁₇CH₃]₂, et/ou le H₂N(CH₂)₃NH(CH₂)₄NH(CH₂)₃NHCH₂COArgN[(CH₂)₁₇CH₃]₂ .Il peut également s'agir de lipides cationiques incorporant un ou plusieurs groupements guanidinium et/ou amidinium, comme plus particulièrement ceux décrits par J.M. LEHN et al. ( Proc. Natl. Acad. Sci. U.S.A, 1996, 93, 9682-9686).

Selon la présente invention, le polymère cationique susceptible d'être mis en oeuvre à titre d'agent de transfection cationique est de préférence un composé de formule générale (I) comme suit : 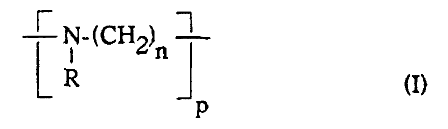 dans laquelle R peut être un atome d'hydrogène ou un groupement de formule : 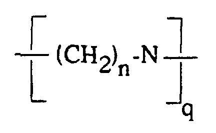 n étant un nombre entier compris entre 2 et 10, et p et q étant des nombres entiers, étant entendu que la somme p+q est telle que le poids moléculaire moyen du polymère est compris entre 100 et 10⁷ Da.

Il est entendu que, dans la formule (I) ci-dessus, la valeur de n peut varier entre les différents motifs p. Ainsi, la formule (I) regroupe à la fois les homopolymères et les hétéropolymères.

Plus préférentiellement, dans la formule (I), n est compris entre 2 et 5. En particulier, les polymères de polyéthylène imine (PEI) et de polypropylène imine (PPI) présentent des propriétés tout à fait avantageuses. Les polymères préférés pour la mise en oeuvre de la présente invention sont ceux dont le poids moléculaire est compris entre 10³ et 5.10⁶. A titre d'exemple, on peut citer le polyéthylène imine de poids moléculaire moyen 50 000 Da (PEI50K), le polyéthylène imine de poids moléculaire moyen 22 000 Da (PEI22K) ou le polyéthylène imine de poids moléculaire moyen 800 000 Da (PEI800K).

Les PEI50K, le PEI22K et le PEI800K sont accessibles commercialement. Quant aux autres polymères représentés par la formule générale (I), ils peuvent être préparés selon le procédé décrit dans la demande de brevet FR 94/08735.

Dans les compositions de la présente invention, l'acide nucléique complexé avec l'agent de transfection cationique peut être aussi bien un acide désoxyribonucléique qu'un acide ribonucléique. Il peut s'agir de séquences d'origine naturelle ou artificielle, et notamment d'ADN génomique, d'ADNc, d'ARNm, d'ARNt, d'ARNr, de séquences hybrides ou de séquences synthétiques ou semi-synthétiques d'oligonucléotides modifiés ou non. Ces acides nucléiques peuvent être d'origine humaine, animale, végétale, bactérienne, virale, etc. Ils peuvent être obtenus par toute technique connue de l'homme du métier, et notamment par criblage de banques, par synthèse chimique, ou encore par des méthodes mixtes incluant la modification chimique ou enzymatique de séquences obtenues par criblage de banques. Ils peuvent être modifiés chimiquement.

Concernant plus particulièrement les acides désoxyribonucléiques, ils peuvent être simple ou double brin de même que des oligonuléotides courts ou des séquences plus longues. Ces acides désoxyribonucléiques peuvent porter des gènes thérapeutiques, des séquences régulatrices de la transcription ou de la réplication, des séquences antisens modifiées ou non, des régions de liaison à d'autres composants cellulaires, etc.

Au sens de l'invention, on entend par gène thérapeutique notamment tout gène codant pour un produit protéique ayant un effet thérapeutique. Le produit protéique ainsi codé peut être une protéine, un peptide, etc. Ce produit protéique peut être homologue vis-à-vis de la cellule cible (c'est-à-dire un produit qui est normalement exprimé dans la cellule cible lorsque celle-ci ne présente aucune pathologie). Dans ce cas, l'expression d'une protéine permet par exemple de pallier une expression insuffisante dans la cellule ou l'expression d'une protéine inactive ou faiblement active en raison d'une modification, ou encore de surexprimer ladite protéine. Le gène thérapeutique peut aussi coder pour un mutant d'une protéine cellulaire, ayant une stabilité accrue, une activité modifiée, etc. Le produit protéique peut également être hétérologue vis-à-vis de la cellule cible. Dans ce cas, une protéine exprimée peut par exemple compléter ou apporter une activité déficiente dans la cellule, lui permettant de lutter contre une pathologie, ou stimuler une réponse immunitaire.

Parmi les produits thérapeutiques au sens de la présente invention, on peut citer plus particulièrement les enzymes, les dérivés sanguins, les hormones, les lymphokines : interleukines, interférons, TNF, etc (FR 92/03120), les facteurs de croissance, les neurotransmetteurs ou leurs précurseurs ou enzymes de synthèse, les facteurs trophiques : BDNF, CNTF, NGF, IGF, GMF, aFGF, bFGF, NT3, NT5, HARP/pléiotrophine, etc la dystrophine ou une minidystrophine (FR 91/11947), la protéine CFTR associée à la mucoviscidose, les gènes suppresseurs de tumeurs : p53, Rb, Rap1A, DCC, k-rev, etc (FR 93/04745), les gènes codant pour des facteurs impliqués dans la coagulation : Facteurs VII, VIII, IX, les gènes intervenant dans la réparation de l'ADN, les gènes suicides (thymidine kinase, cytosine déaminase), les gènes de l'hémoglobine ou d'autres transporteurs protéiques, les gènes correspondant aux protéines impliquées dans le métabolisme des lipides, de type apolipoprotéine choisie parmi les apolipoprotéines A-I, A-II, A-IV, B, C-I, C-II, C-III, D, E, F, G, H, J et apo(a), les enzymes du métabolisme comme par exemple la lipoprotéine lipase, la lipase hépatique, la lécithine cholestérol acyltransférase, la 7 alpha cholestérol hydroxylase, la phosphatidique acide phosphatase, ou encore des protéines de transfert de lipides comme la protéine de transfert des esters de cholesterol et la protéine de transfert des phospholipides, une protéine de liaisons des HDL ou encore un récepteur choisi par exemple parmi les récepteurs LDL, récepteurs des chylomicrons-remnants et les récepteurs scavenger, etc.

L'acide nucléique thérapeutique peut également être un gène ou une séquence antisens, dont l'expression dans la cellule cible permet de contrôler l'expression de gènes ou la transcription d'ARNm cellulaires. De telles séquences peuvent, par exemple, être transcrites dans la cellule cible en ARN complémentaires d'ARNm cellulaires et bloquer ainsi leur traduction en protéine, selon la technique décrite dans le brevet EP 140 308. Les gènes thérapeutiques comprenent également les séquences codant pour des ribozymes, qui sont capables de détruire sélectivement des ARN cibles (EP 321 201).

Comme indiqué plus haut, l'acide nucléique peut également comporter un ou plusieurs gènes codant pour un peptide antigénique, capable de générer chez l'homme ou l'animal une réponse immunitaire. Dans ce mode particulier de mise en oeuvre, l'invention permet donc la réalisation soit de vaccins soit de traitements immunothérapeutiques appliqués à l'homme ou à l'animal, notamment contre des microorganismes, des virus ou des cancers. Il peut s'agir notamment de peptides antigéniques spécifiques du virus d'Epstein Barr, du virus HIV, du virus de l'hépatite B (EP 185 573), du virus de la pseudo-rage, du "syncitia forming virus, d'autres virus ou encore spécifiques de tumeurs (EP 259 212).

Préférentiellement, l'acide nucléique comprend également des séquences permettant l'expression du gène thérapeutique et/ou du gène codant pour le peptide antigénique dans la cellule ou l'organe désiré. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus. A cet égard, on peut citer par exemple les promoteurs des gènes E1A, MLP, CMV, RSV, etc. En outre, ces séquences d'expression peuvent être modifiées par addition de séquences d'activation, de régulation, etc. Il peut aussi s'agir de promoteur, inductible ou répressible.

Par ailleurs, l'acide nucléique peut également comporter, en particulier en amont du gène thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle. L'acide nucléique peut également comporter une séquence signal dirigeant le produit thérapeutique synthétisé vers un compartiment particulier de la cellule.

Dans un autre mode de mise en oeuvre, les compositions revendiquées peuvent comprendre en outre un adjuvant de type dioleoylphosphatidyléthanolamine (DOPE), l'oléoylpalmitoylphosphatidyléthanolamine (POPE), les di-stéaroyl, -palmitoyl, mirystoyl phosphatidyléthanolamines ainsi que'leurs dérivé N-méthylés 1 à 3 fois, les phosphatidylglycérols, les diacylglycérols, les glycosyldiacylglycérols, les cérébrosides (tels que notamment les galactocérébrosides), les sphingolipides (tels que notamment les sphingomyélines) ou encore les asialogangliosides (tels que notamment les asialoGM1 et GM2).

Très récemment, la demanderesse a démontré qu'il était également particulièrement avantageux d'employer à titre d'adjuvant dans des compositions transfectantes, un composé intervenant, directement ou non, au niveau de la condensation d'acides nucléiques. Ce composé est constitué, en tout ou partie, de motifs peptidiques (KTPKKAKKP) et/ou (ATPAKKAA), le nombre des motifs pouvant varier entre 2 et 10. Un tel agent peut également dériver d'une partie d'une histone, d'une nucléoline, d'une protamine et/ou de l'un de leurs dérivés (WO 96/25508). Un tel composé peut être avantageusement incorporé dans la composition revendiquée.

Les compositions selon l'invention peuvent également mettre en oeuvre un ou plusieurs éléments de ciblage permettant de diriger les complexes nucléiques vers des recepteurs ou des ligands à la surface de la cellules. A titre d'exemple, la composition de la présente invention peut comprendre un ou plusieurs anticorps dirigés contre des molécules de la surface cellulaire, ou encore un ou plusieurs ligands de recepteurs membranaires comme l'insuline, la transferrine, l'acide folique ou tout autre facteur de croissance, cytokines ou vitamines. Avantageusement, la composition peut utiliser des lectines, modifiées ou non, afin de cibler des polysaccharides particuliers à la surface de la cellule ou sur la matrice extracellulaire avoisinante. Des protéines à motif RGD, des peptides contenant un tandem de motifs RGD, cyclique ou non, ainsi que des peptides polylysine peuvent ainsi être utilisés. Plus récemment, il a également été décrit des peptides ligands, naturels ou synthétiques, intéressants notamment pour leur sélectivité vis à vis de cellules spécifiques et capables de promouvoir efficacement l'internalisation au niveau de ces cellules. (Bary et al. Nature Medicine, 2, 1996, 299-305). Ces agents de ciblages sont généralement conjugués à l'agent de transfection cationique considéré.

La présente invention vise également un procédé de préparation des compositions revendiquées. Plus précisément, elle se rapporte à un procédé de préparation d'une composition comprenant des particules de complexes agent(s) de transfection cationique(s)/acides nucléiques, stabilisées en taille, caractérisé en ce que l'agent transfectant et l'acide nucléique sont mis en contact en présence d'une quantité suffisante en agent de surface non ionique pour stabiliser les particules de complexes nucléiques ainsi formées à une taille inférieure à environ 160 nm.

Plus précisément, l'un des composants à savoir l'acide nucléique ou le lipofectant est au préalable mélangé à l'agent de surface non ionique avant d'être mis en présence avec le second composant. On prévient ainsi la manifestation de tout phénomène d'aggrégation qui spontanément se serait manifesté en absence dudit agent de surface non ionique.

Les compositions selon l'invention peuvent être formulées en vue d'administrations par voie topique, cutanée, orale, rectale, vaginale, parentérale, intranasale, intraveineuse, intramusculaire, sous-cutanée, intraoculaire, transdermique, etc. De préférence, les compositions pharmaceutiques de l'invention contiennent un véhicule pharmaceutiquement acceptable pour une formulation injectable, notamment pour une injection directe au niveau de l'organe désiré, ou pour une administration par voie topique (sur peau et/ou muqueuse). Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Les doses d'acide nucléique utilisées pour l'injection ainsi que le nombre d'administrations peuvent être adaptés en fonction de différents paramètres, et notamment en fonction du mode d'administration utilisé, de la pathologie concernée, du gène à exprimer, ou encore de la durée du traitement recherchée. En ce qui concerne plus particulièrement le mode d'administration, il peut s'agir soit d'une injection directe dans les tissus ou les voies circulatoires, soit d'un traitement de cellules en culture suivi de leur réimplantation in vivo, par injection ou greffe.

Les compositions selon l'invention sont particulièrement avantageuses sur le plan thérapeutique. On peut désormais envisager selon la présente invention d'administrer efficacement des complexes acides nucléiques de taille convenable et de rapport de charge réduit, ce qui est particulièrement bénéfique sur le plan *in vivo*. Les interactions sérum/complexes nucléiques généralement observées sont avec les compositions revendiquées significativement atténuées.

La présente invention sera plus complètement décrite à l'aide des exemples et figures qui suivent, qui doivent être considérés comme illustratifs et non limitatifs.

### FIGURES

Figure 1: Effet du poloxalkol sur l'évolution de la taille des complexes RPR 120535/ADN, au rapport de charge (+/-) 2,5, en fonction du temps.
Figure 2 : Effet du poloxalkol sur l'évolution de la taille des complexes RPR 120531/ADN, au rapport de charge (+/-) 2,5 en fonction du temps.
Figure 3 : Effet du poloxalkol sur les associations RPR 120535/ADN.
Figure 4 : Effet du poloxalkol sur l'activité luciférase (RLU/5µl de lysat) des complexes RPR 120535/ADN, au rapport de charge (+/-) 2,5.
Figure 5 : PARTIE A : Représentation schématique de la structure des complexes RPR 120535/ADN en l'absence ou en présence de poloxalkol.
   PARTIE B : Représentation schématique de la structure des complexes BGTC/ADN en l'absence et en présence de poloxalkol

### MATERIELS

L'agent de surface mis en oeuvre dans les exemples ci-après est le poloxalkol de formule OH(CH₂CH₂O)₇₅(CH(CH₃)CH₂O)₃₀(CH₂CH₂O)₇₅H. Il est commercialisé sous la marque Pluronic F68® . La solution mère de poloxalkol mise en oeuvre dans les exemples qui suivent est à 10 % (poids/volume) dans l'eau.

L'ADN utilisé pour réaliser les échantillons est le plasmide de pXL2774 décrit dans le PCT/FR 96/01414. Il est utilisé à une concentration de 0,7 mg/ml dans un tampon Tris/EDTA (10 mM/0,1 mM) de pH 7,5.

Les lipides cationiques utilisés sont les suivants :
H₂N(CH₂)₃NH(CH₂)₄NH(CH₂)₃NHCH₂COGlyN[(CH₂)₁₇CH₃]₂ (RPR 120535) (sel d'acétate) et H₂N(CH₂)₃NH(CH₂)₄NH(CH₂)₃NHCH₂COArgN[(CH₂)₁₇CH₃] (RPR 120531) (sel d'acide trifluoroacétique), tous deux décrits dans le PCT/FR 96/01774. Ils sont utilisés à une concentration de 5 mM dans de l'eau où ils sont solubilisés par chauffage à 50°C pendant 25 minutes, les solutions étant ensuite refroidies à température ambiante.

### METHODES

La mesure du diamètre hydrodynamique est réalisée avec un Coulter N4Plus, en utilisant des cuves plastiques (quatre faces transparentes) remplies avec 800 µl des différentes solutions, la mesure étant réalisée à 90°C en mode unimodale.

Les mesures de fluorescence sont réalisées sur Perkin Elmer LS50B, en utilisant des longueurs d'onde d'excitation et d'émission respectivement de 260 nm et 590 nm. Les largeurs de fentes pour l'excitation et l'émission sont réglées à 5 nm. La valeur de fluorescence est enregistrée après ajout de 5 µg de Bromure d'étidium/ml en concentration finale.

Les expériences de cryomicroscopie électronique à transmission (cryo-TEM) sont réalisées avec 7 µl d'échantillons préparés à 0,5 mg d'ADN/ml, qui sont disposés sur une grille en cuivre carbonée recouverte d'une membrane à trous. Les grilles sont ensuite plongées dans de l'éthane liquide de manière à transformer l'eau liquide en eau vitreuse. Puis, cette grille est installée dans un porte-objet refroidi par de l'azote liquide, et on introduit dans le microscope (Philips CM12) pour visualisation.

Les expériences de diffusion de rayons X aux petits angles sont réalisées au synchrotron (LURE) à Orsay (France) sur la ligne D43. Les échantillons sont préparés à 0,5 mg ADN/ml, puis centrifugés. Les culots sont disposés dans une cellule dont les deux fenêtres sont constituée de kapton. Un monochromateur au Germanium (111 réflection) permet la sélection de la longueur d'onde de 0,138 nm.

Les transfections *in vivo* sont réalisées en injectant 200 µl, dans la veine de la queue de souris agées de 30 jours, d'une solution contenant 0,2 mg ADN/ml associé avec un vecteur lipidique cationique. 24 heures après l'injection, les souris sont euthanasiées, puis différents organes sont récupérés (poumon, foie, coeur, rein, rate). Ces organes sont ensuite broyés dans un tampon de lyse à l'aide d'un ultraturax. Les broyats obtenus sont ensuite centrifugés, puis 10 µl du surnageant est prélevé afin de doser l'activité luciférase.

### EXEMPLE 1 : Influence du poloxalkol sur la taille des complexes RPR 120535/ADN.

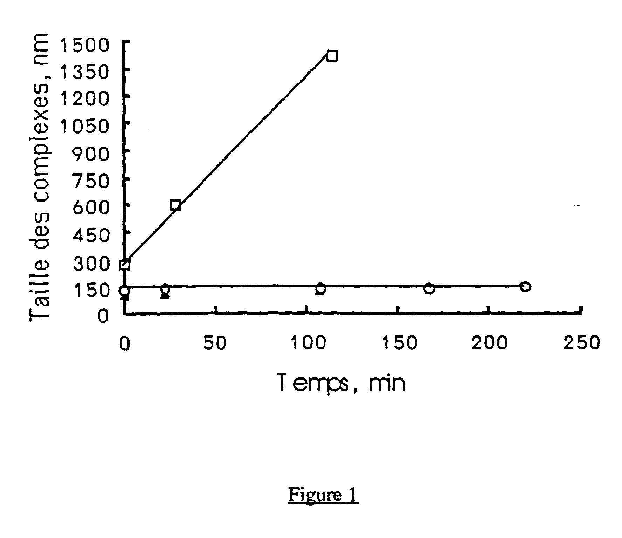

Le plasmide (10 µg/ml) est mis dans une solution contenant 150 mM de NaCl et différentes concentration en poloxalkol. Le lipofectant RPR 120535 est ensuite ajouté pour obtenir un rapport de charge (+/-) 2,5. La taille des complexes est ensuite mesurée par spectroscopie de corrélation de photons selon le protocole décrit dans Méthodes. Les résultats sont présentés sur la figure 1.

On note qu'en absence de poloxalkol (□), la taille des particules évolue de 274 nm jusqu'à un maximum de 1000 nm en quelques dizaine de minutes. Puis on observe un précipité après quelques heures d'incubation.
En revanche, en présence de 0,1 % (+) de poloxalkol, on observe une diminution de la taille des complexes, taille qui demeure stable pour une concentration de 0,8% (○) et 1 % (▲) de poloxalkol et n'évolue pas au delà de 150 nm.

L'incubation du lipofectant seul avec le poloxalkol à 1% (poids/volume) ne forme pas de particules détectables par Diffusion Quasi Elastique de la lumière. De même, lorsque l'ADN est incubé avec le poloxalkol à 1%, aucune particule ne peut être détectée.

### EXEMPLE 2 : Influence de poloxalkol sur la taille des complexes RPR 120531/ADN.

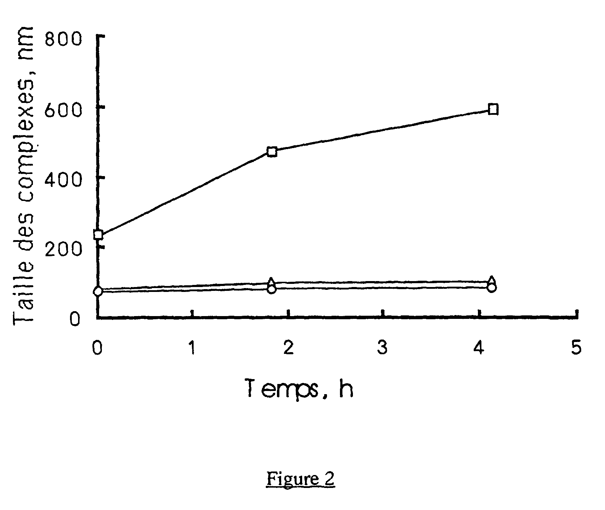

Pour ce faire, l'exemple n°1 a été répété avec le lipide cationique RPR 120531.
Le plasmide (10 µg/ml) est mis dans une solution contenant 150 mM de NaCl à différentes concentration en poloxalkol. Le RPR 120531 est ensuite ajouté pour obtenir un rapport de de charge (+/-) 2,5. La taille des complexes est mesurée par spectroscopie de corrélation de photons (Coulter N4plus). Les résultats sont présentés sur la figure 2.
On note qu'en l'absence de poloxalkol, la taille des complexes RPR 120531/ADN évolue de 234 nm à 590 nm en quelques heures alors qu'elle demeure stable en présence de 0,5% (▲) et 0,9% (○) de poloxalkol.

### EXEMPLE 3 : Contrôle du degré de compaction ADN/lipofectant en présence et en l'absence de poloxalkol.

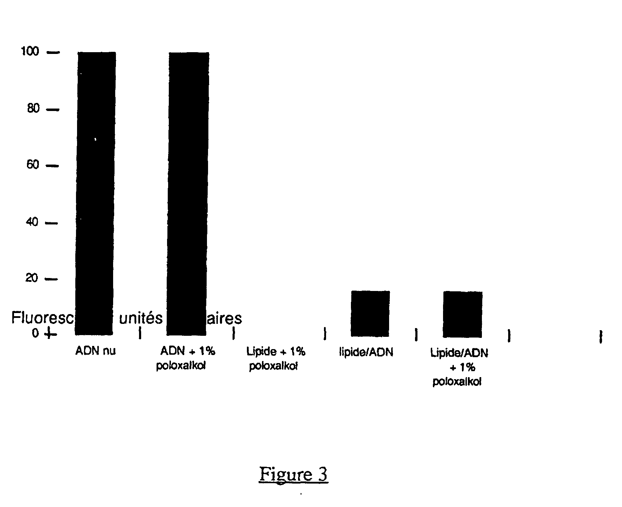

Il a été vérifié que les particules obtenues en présence de poloxalkol étaient bien des particules issues de l'association de l'ADN avec le lipide cationique. A ces fins, des expériences d'insertion d'une sonde, qui fluoresce lorsqu'elle est intercalée dans l'ADN ont été réalisées. Le niveau de fluorescence est important lorsque l'ADN est libre et en revanche faible lorsque celui-ci est inaccessible car compacté au lipide cationique. La figure 3 rend compte des résultats obtenus.
On observe les niveaux de fluorescence obtenus après association de l'ADN au lipide cationique en présence et en absence de poloxalkol. Tout d'abord on note que la fluorescence du bromure d'éthidium intercalé à l'ADN n'est pas modifié par la présence ou l'absence de 1% de poloxalkol. Les niveaux de fluorescence des complexes lipide cationique/ADN (rapport de charde (+/-) 2,5) en présence ou en l'absence de 1% de poloxalkol sont en effet du même ordre. Cette valeur de fluorescence résiduelle indique que l'ADN n'est pas accessible pour la sonde.
En conclusion , le poloxalkol n'empêche donc pas l'association de l'ADN avec le lipide cationique de se faire normalement.

### EXEMPLE 4 : Transfection in vitro avec de l'ADN ou du RPR 120535 contenant 1 % de poloxalkol.

Nous avons voulu vérifier quel était l'effet du poloxalkol lorsqu'il était mélangé à de l'ADN seul ou à du lipide cationique seul. Pour cela les deux échantillons suivants ont été préparés :
Echantillon 1 : 10 µg ADN/ml dans un milieu contenant 300 mM de NaCl et 1% (poids/volume) de poloxalkol.
Echantillon 2 : 60 µM de RPR 120535 dans un milieu contenant 300 mM de NaCl et 1% poloxalkol.

Ces échantillons sont préparés en mélangeant dans l'ordre pour l'échantillon 1 : eau, NaCl, ADN et poloxalkol, et pour l'échantillon 2 : eau, NaCl, poloxalkol et RPR 120535 dans les quantités figurant dans le tableau I ci-dessous :

**TABLEAU I**

| Essai | H₂0 | NaCl (5M) | ADN (0,7 mg/ml) | poloxalkol (10%) | RPR 120535 (5 mmol) |
|---|---|---|---|---|---|
| 1 (µl) | 413 | 30 | 7,1 | 50 | 0 |
| 2 (µl) | 414 | 30 | 0 | 50 | 6 |

L'activité biologique de ces deux échantillons est testée sur des cellules NIH 3T3. Un volume de 50 µl des différents échantillons est déposé par puits sur plaque de 24 puits.
La transfection est réalisée en l'absence de sérum de veau foetal. Ce dernier est ajouté 2 heures après l'addition des complexes.
Les résultats indiquent qu'aucun transfert de gène n'est observé avec l'ADN non-complexé en présence de 1% de poloxalkol et en l'absence de 10% de sérum de veau foetal.
D'autre part le dosage des protéines totales démontre qu'il n'y a pas de toxicité apparente.

### EXEMPLE 5 : Transfection in vitro avec des solutions de complexes lipofectant RPR 120535/ADN stabilisées à différentes concentrations en poloxalkol.

On a cherché à apprécier l'incidence de la stabilisation des particules par le poloxalkol sur l'efficacité de transfection de ces particules en présence de poloxalkol.
Pour se faire, des échantillons contenant différentes concentrations en poloxalkol ont été préparés.
Quatre échantillons de rapport de charge (+/-) 2,5 sont préparés dans un milieu contenant 300 mM de NaCl à différentes concentrations en poloxalkol (0; 0,5%; 0,8% et 1%; poids/volume) :
Echantillon 1 : rapport de charge (+/-) 2,5, 300 mM de NaCl.
Echantillon 2 : rapport de charge (+/-) 2,5, 300 mM de NaCl, et 0,5% de poloxalkol.
Echantillon 3 : rapport de charge (+/-) 2,5, 300 mM de NaCl, et 0,8% de poloxalkol.
Echantillon 4 : rapport de charge (+/-) 2,5, 300 mM de NaCl, et 1% de poloxalkol.
Ces échantillons ont été préparés en mélangeant dans l'ordre : eau, NaCl, ADN, poloxalkol et RPR 120535, dans les quantités figurant en tableau II ci-dessous :

**TABLEAU II**

| | | | | | |
|---|---|---|---|---|---|
| Essai | H₂O | NaCl (5 M) | ADN (0,7 mg/ml) | poloxalkol (10%) | RPR 120535 (5 mM) |
| 1 (µl) | 737 | 48 | 11,4 | 0 | 4 |
| 2 (µl) | 697 | 48 | 11,4 | 40 | 4 |
| 3 (µl) | 672 | 48 | 11,4 | 64 | 4 |
| 4 (µl) | 657 | 48 | 11,4 | 80 | 4 |

* L'échantillon 1 appartient à une gamme de concentration en lipide cationique où il n'y a pas de stabilité des particules en solution, c'est-à-dire que les particules s'agrègent les unes aux autres pour former de larges paquets d'agrégats qui possèdent des diamètres hydrodynamique important (supérieur à 1000 nm).
* L'échantillon 2 a le même rapport de charge lipide cationique/ADN que l'échantillon 1, mais le mélange est réalisé en présence de 0,5 % (poids/volume) de poloxalkol. Cependant cette concentration n'est pas suffisante pour assurer une stabilisation complète des particules (cf exemple n°1 : influence d'un polymère non-ionique sur la taille des complexes RPR 120535/ADN).
* Les échantillons 3 et 4 sont stables, le diamètre hydrodynamique des particules restent aux alentours de 150 nm.

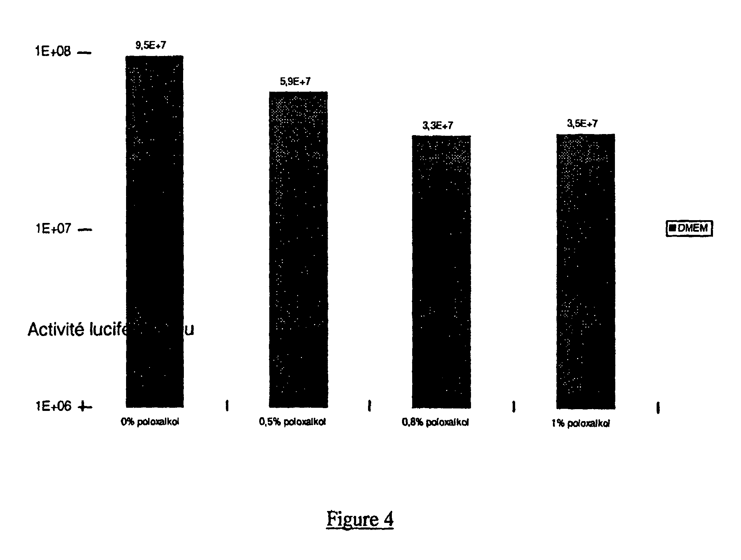

L'activité biologique des différentes formulations est testée sur des cellules NIH 3T3. Un volume de 50 µl des différents échantillons (à 10 µg ADN/ml) est ajouté par puits. La transfection est réalisée en l'absence de sérum de veau foetal. Ce dernier est ajouté 2 heures après l'addition des complexes. Les résultats obtenus sont présentés sur la figure 4.

On note en figure 4 que le poloxalkol, quelque soit sa concentration, n'altère pas la transfection *in vitro* des cellules NIH 3T3. Le poloxalkol ne modifie pas les propriétés de transfection en l'absence de sérum de veau foetal des complexes RPR 120535/ADN au rapport de charge (+/-) 2,5.

### EXEMPLE 6 : Détermination de la structure des complexes RPR 120535/ADN, BGTC/ADN, et BGTC/DOPE/ADN stabilisés par du poloxalkol.

Dans cette étude, nous avons déterminé par diffusion de rayons X aux petits angles et par microscopie électronique à transmission la structure des complexes transfectant lipide cationique/ADN.

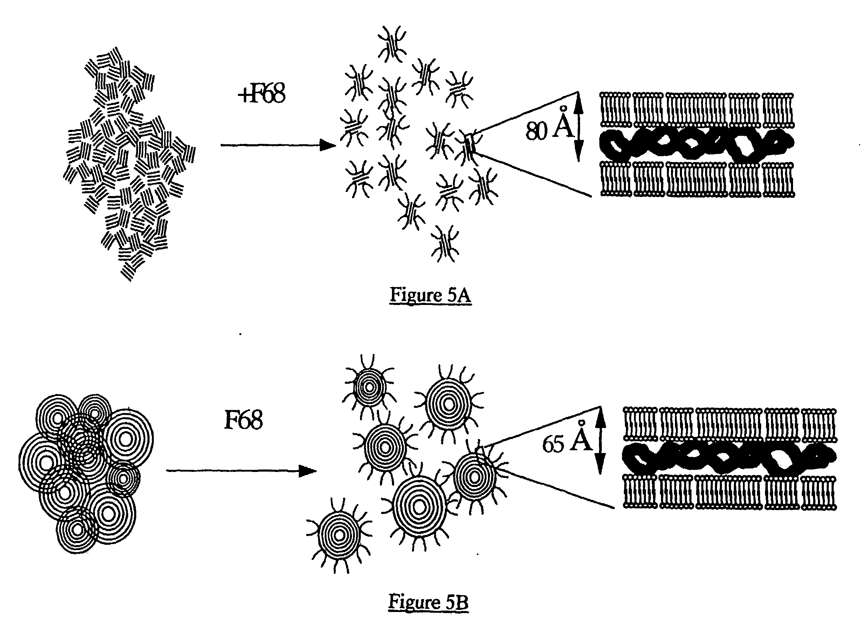

La figure 5A concerne les complexes RPR 120535/ADN. La structure obtenue en l'absence et en présence de poloxalkol est une structure lamellaire dans laquelle l'ADN est pris en sandwich entre des bicouches lipidiques dont la périodicité est de 8 nm. La présence de poloxalkol permet d'obtenir des complexes de petite taille, alors qu'en l'absence de poloxalkol, nous obtenons des précipités dont la taille est supérieure à 1000 nm.
La figure 5B représente les structures obtenues avec le lipide cationique BGTC (décrit dans la demande de brevet WO 97/31935). Dans ce cas, nous obtenons également une structure lamellaire dont la périodicité est de 6,5 nm en présence ou en l'absence de poloxalkol. La présence de poloxalkol permet également dans ce cas de disposer de complexes de petite taille. Les résultats sont obtenus invariablement pour des complexes contenant ou non l'adjuvant DOPE.
Nous pouvons donc en conclure que l'ajout d'un poloxalkol ne gène pas l'association de l'ADN au lipide cationique, mais ne modifie que l'état colloïdal de ce complexe.

### EXEMPLE 7 : Transfection in vivo après injection systémique de complexes lipide cationique/ADN stabilisés par du poloxalkol.

### 1) RPR 120535/ADN/F68®

Nous avons comparé les efficacités de transfert de gène après injections systémiques de complexes RPR 120535/ADN précipités, et de ces mêmes complexes stabilisés par l'addition de Pluronic F68® . Le tableau III ci-dessous indique que lorsque les complexes sont réalisés en l'absence de F68® , on retrouve uniquement une expression dans les poumons. Par contre, lorsque les particules sont stabilisées colloïdalement par l'ajout de F68® , l'activité luciférase est augmentée d'un facteur 10 dans les poumons, et on retrouve une expression dans d'autres tissus (foie, coeur).

**TABLEAU III**

| Lipide | RPR 120535 | |
|---|---|---|
| Formulation | 0% de F68 | 10% de F68 |
| Souris | balbc. | balbc |
| Poumons | 2 ± 0,9 | 14 ± 0,9 |
| foie | 0 | 2 ± 3 |
| Reins | 0 | 0 |
| Coeur | 0 | 1 ± 0,4 |

### 2) BGTC/DOPE/ADN/F68®

Des expériences identiques à celles décrites ci-dessus ont été réalisées en utilisant un autre lipide : le BGTC. Des résultats similaires ont été obtenus, à savoir une amélioration du transfert de gène dans les poumons et une expression mesurable dans le foie, le coeur, et les reins. Ces résultats ont été obtenus avec différentes races de souris (balbc, C57B16, et C57B16 déficientes en Apolipoprotéine E).Il est remarquable que des niveaux de transfection identiques soient obtenus avec des souris C57B16 normales ou déficientes en Apolipoprotéine E, car ces dernières possèdent un taux de lipide très important, c'est-à-dire dix fois plus qu'une souris normale. En effet, étant donné que l'on utilise des vecteurs non-viraux lipidiques, on aurait pu s'attendre à ce que ces complexes soient déstabilisés par la présence de ces lipides endogènes en grande quantité. Les résultats de transfection *in vivo* de complexes BGTC/DOPE/ADN en l'absence ou en présence de poloxalkol sont rassemblés dans le tableau IV ci-dessous :

**TABLEAU IV**

| Lipide | BGTC/DOPE | | | |
|---|---|---|---|---|
| Formulation | 0% F68 | 4% F68 | | |
| Souris | balbc | balbc | C57B16 | C57B16 KoApoE |
| Poumons | 6.7±2,8 | 86,5±65,4 | 220±99 | 201±12 |
| foie | 0 | 11,4+11,5 | 47±26 | 47±23 |
| Reins | 0 | 0,34±0,16 | 1,8±0,9 | 2,2±0,1 |
| Coeur | 0 | 0,74±1,05 | 0,73±0,7 | 0,6±0,0 |

### EXEMPLE 8 : Utilisation d'autres agents de surface non-ioniques.

D'autres agents de surface non-ioniques ont été testés : dendrimère polyéthylène glycol, polyoxyéthylène alcool, polyoxyéthylène nonylphényléther. Ils montrent la même capacité que le F68® à stabiliser les complexes lipides cationiques/ADN. Le lipide cationique avec lequel les études ont été réalisées est le RPR 120535.

### 1) dendrimères polyéthylène glycol

Nous avons utilisé un dendrimère possédant 1 tête de polyéther benzylique de génération 2 sur laquelle est greffé du polyéthylène glycol (PEG) de masse moléculaire 5000 Da (dénommé SAS11), et un autre dendrimère du même type, mais contenant 2 têtes de polyéther benzylique greffées sur un PEG de masse 11 000 Da (dénommé SAS9). Le tableau IV ci-dessous indique qu'avec ces dendrimères, dès 0,02% (poids/volume), nous n'obtenons plus de précipité micronique de complexes transfectants, mais plutôt des particules de diamètre inférieur à ou de l'ordre de 100 nm.

### 2) polyoxyéthylène alcool

Ce produit encore dénommé "Brij" comportant 100 oxyéthylène pour la partie hydrophile, permet d'obtenir au bout d'1 heure des complexes RPR 120535/ADN d'un diamètre inférieur à 100 nm.

### 3) Polyoxyéthylène nonylphényléther

Ce produit permet aussi de stabiliser colloïdalement les complexes transfectants RPR 120535/ADN. Le diamètre des particules est également de l'ordre de 100 nm.

Le tableau V ci-dessous représente le diamètre, en nm, des complexes RPR 120535/ADN, au rapport de charge de 2,75 (+/-) à 0,25 mg d'ADN/ml dans 150 mM de NaCl, stabilisés par différents agents de surface non-ioniques.

**TABLEAU V**

| Polymère (p/v) | 0,05% | 0,1% | 0,2% |
|---|---|---|---|
| Brij 700 | 114 nm | 69 nm | 74 nm |
| SAS11 | 152 nm | 99 nm | 71 nm |
| SAS 9 | 104 nm | 85 nm | 75 nm |
| polyoxyéthylène nonylphényl éther | 142 nm | 132 nm | 111 nm |

Pour l'ensemble des formulations stabilisées avec ces différents agents de surface non-ioniques, nous avons vérifié l'état de condensation de l'ADN avec le lipide cationique par des mesures de fluorescence. Les résultats ont indiqué que la présence de ces surfactants à la surface des complexes lipide cationique/ADN ne modifie pas la condensation de l'ADN avec les lipides cationiques (résultats non-montrés).

## Revendications

1. Composition **caractérisée en ce qu'**elle comprend des particules de complexes lamellaires agent(s) de transfection cationique(s)/acides nucléiques et **en ce qu'**elle comprend au moins un agent de surface non ionique en quantité comprise entre 0,01 % et 10 % poids/volume de façon à obtenir des complexes lamellaires stabilisés à un diamètre inférieur ou égal à 160 nm.

2. Composition selon la revendication 1 **caractérisée en ce que** l'agent de surface comprend au moins un segment hydrophobe et au moins un segment hydrophile.

3. Composition selon la revendication 2 **caractérisée en ce que** le segment hydrophobe est choisi parmi les chaînes aliphatiques, les polyoxyalkylènes, les polyesters d'alkylidène, les polyéthylène glycol à tête polyéther benzylique, et le cholestérol.

4. Composition selon la revendication 2 **caractérisée en ce que** le segment hydrophile est choisi parmi les polyoxyalkylènes, les alcools polyvinyles, les polyvinylpyrrolidones, ou les saccharides.

5. Composition selon l'une des revendications 1 à 4 **caractérisée en ce que** l'agent de surface est un polyoxalkylène de formule générale :
HO(CH₂CH₂O)ₐ(CH(CH₃)CH₂O)_{b}(CH₂CH₂O)_{c}H
avec a, b et c représentant indépendamment l'un de l'autre des nombres entiers pouvant varier entre 20 et 100.

6. Composition selon la revendication 5 **caractérisée en ce qu'**elle contient à titre d'agent de surface un composé de formule générale OH(CH₂CH₂O)ₐ(CH(CH₃)CH₂O)_{b}(CH₂CH₂O)_{c}H. avec a égal à 75, b à 30 et c à 75.

7. Composition selon l'une des revendications 1 à 4 **caractérisée en ce qu'**elle contient à titre d'agent de surface un composé de la famille des polyéthylène glycol à tête polyéther benzylique dendritique.

8. Composition selon l'une des revendications 1 à 4 **caractérisée en ce qu'**elle contient à titre d'agent de surface un composé de la famille des polyoxyéthylène alcooL

9. Composition selon l'une des revendications 1 à 4 **caractérisée en ce qu'**elle contient à titre d'agent de surface le polyoxyéthylène nonylphényléther.

10. Composition selon la revendications 1 **caractérisée en ce que** l'agent de surface est présent en quantité comprise entre 0,02% et 5% poids/volume.

11. Composition selon la revendication 1 **caractérisée en ce que** l'agent de transfection cationique est un lipofectant.

12. Composition selon la revendication 11 **caractérisée en ce que** le lipofectant est une molécule amphiphile comprenant au moins une région lipophile associée ou non à une région hydrophile.

13. Composition selon l'une des revendications 11 ou 12 **caractérisée en ce qu'**il s'agit d'un lipofectant comprenant au moins une région polyamine de formule générale :
H₂N-(-(CH)ₘ-NH-)ₙ-H
dans laquelle m est un nombre entier supérieur ou égal à 2 et n est un nombre entier supérieur ou égal à 1, m pouvant varier entre les différents groupes de carbone compris entre 2 amines, associée de manière covalente à une région lipophile de type chaîne hydrocarbonée, saturée ou non, du cholestérol, ou un lipide naturel ou synthétique capable de former des phases lamellaires ou héxagonales.

14. Composition selon la revendication 13 **caractérisée en ce que** la région polyamine est représentée par la spermine ou un de ses analogues ayant conservé ses propriétés de liaison à l'acide nucléique.

15. Composition selon l'une des revendications 11 ou 12 **caractérisée en ce qu'**il s'agit d'un lipofectant de formule générale : 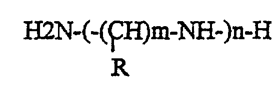 dans laquelle R figurant la région lipophile est représenté par la formule générale : 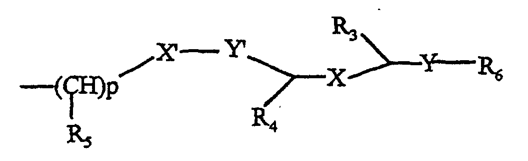 dans laquelle X et X' représentent, indépendamment l'un de l'autre, un atome d'oxygène, un groupement méthylène -(CH₂)_{q}- avec q égal à 0, 1, 2 ou 3, ou un groupement amino, -NH- ou -NR'- avec R' représentant un groupement alkyle en C₁ à C₄, Y et Y' représentent indépendamment l'un de l'autre un groupement méthylène, un groupement carbonyle ou un groupement C=S, R₃, R₄ et R₅ représentent indépendamment l'un de l'autre un atome d'hydrogène ou un radical alkyle, substitué ou non, en C₁ à C₄, avec p pouvant varier entre 0 et 5, R₆ représente un dérivé du cholestérol ou un groupement alkyle amino -NR₁R₂ avec R₁ et R₂ représentant indépendamment l'un de l'autre un radical aliphatique, saturé ou non, linéaire ou ramifié en C₁₂ à C₂₂.

16. Composition selon l'une des revendications 11 ou 12 **caractérisée en ce qu'**il s'agit d'un lipofectant de formule générale : 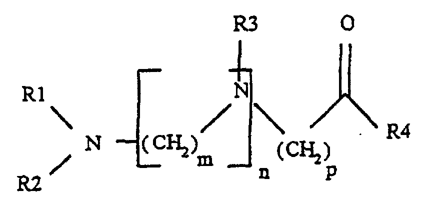 dans laquelle R₁, R₂ et R₃ représentent indépendamment l'un de l'autre un atome d'hydrogène ou un groupement -(CH₂)_{q}-NRR' avec q pouvant varier entre 1, 2, 3, 4, 5 et 6 ceci de manière indépendante entre les différents groupements R₁, R₂ et R₃, et R et R' représentant indépendamment l'un de l'autre un atome d'hydrogène ou un groupement -(CH₂)_{q}'-NH₂, q' pouvant varier entre 1, 2, 3, 4, 5 et 6 ceci de manière indépendante entre les différents groupements R et R', m, n et p représentent, indépendamment l'un de l'autre, un nombre entier pouvant varier entre 0 et 6 avec lorsque n est supérieur à 1, m pouvant prendre des valeurs différentes et R₃ des significations différentes au sein de la formule générale précédente, et R₄ représente un groupement de formule générale : 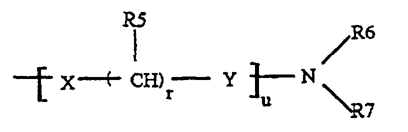 dans laquelle R₆ et R₇ représentent indépendamment l'un de l'autre un atome d'hydrogène ou un radical aliphatique, saturé ou non, en C₁₀ à C₂₂ avec au moins l'un des deux groupements étant différent de l'hydrogène, u est un nombre entier choisi entre 0 et 10 avec lorsque u est un entier supérieur à 1, R₅, X, Y et r pouvant avoir des significations différentes au sein des différents motifs [ X-(CHR₅)ᵣ-Y], X représente un atome d'oxygène, de soufre ou un groupement amine monoalkylé ou non, Y représente un groupement carbonyle ou un groupement méthylène, R₅ représente un atome d'hydrogène ou une chaîne latérale d'acide aminé naturel, le cas échéant substituée, et r représente un entier variant entre 1 et 10 avec lorsque r est égal à 1, R₅ représentant une chaîne latérale d'acide aminé naturel substitué ou non, et lorsque r est supérieur à 1, R₅ représentant un atome d'hydrogène.

17. Composition selon l'une des revendications 11 ou 12 **caractérisé en ce que** ledit lipofectant est un lipide cationique porteur d'un ou plusieurs groupements guanidinium et/ou amidinium.

18. Composition selon l'une des revendications 1 à 10 **caractérisée en ce que** l'agent de transfection cationique est un polymère cationique.

19. Composition selon la revendication 18 **caractérisée en ce que** ledit polymère cationique est un composé de formule générale (I): 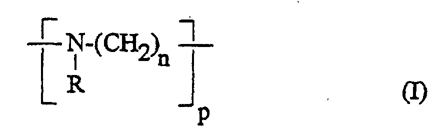 dans laquelle R peut être un atome d'hydrogène ou un groupe de formule: 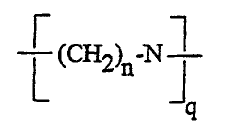 n est un nombre entier compris entre 2 et 10, et p et q sont des nombres entiers, étant entendu que la somme p+q est telle que le poids moléculaire moyen du polymère est compris entre 100 et 10⁷ Da.

20. Composition selon l'une des revendications 18 ou 19 **caractérisée en ce qu'**il s'agit du polyéthylène imine de poids moléculaire moyen 50 000 Da (PEI50K), du polyéthylène imine de poids moléculaire moyen 22 000 Da (PEI22K), ou le polyéthylène imine de poids moléculaire moyen 800 000 Da (PEI800K).

21. Composition selon l'une des revendications 1 à 11 **caractérisée en ce que** l'agent de transfection cationique est de préférence choisi parmi la lipofectamine, la dioctadécylamidoglycyl spermine (DOGS) la 5-carboxyspermylamide de la palmitoylphosphatidylethanolamine (DPPES), le (Dioctadécyl-carbamoylméthoxy)-acétate de 2-5-bis-(3-amino-propylamino)-pentyle ou le (Dioctadécyl-carbamoylméthoxy)-acétate de 1,3-bis-(3-amino-propylamino)-2 propyle, le {H₂N(CH₂)₃}₂N(CH₂)₄N{(CH₂)₃NH₂}(CH₂)₃NHCH₂COGlyN[(CH₂)₁₇CH₃]₂, le H₂N(CH₂)₃NH(CH₂)₄NH(CH₂)₃NHCH₂COGlyN[(CH₂)₁₇CH₃]₂, et le H₂N(CH₂)₃NH(CH₂)₄NH(CH₂)₃NHCH₂COArgN[(CH₂)₁₇CH₃]₂.

22. Composition selon la revendication 1 **caractérisée en ce que** l'acide nucléique est un acide désoxyribonucléique.

23. Composition selon la revendication 1 **caractérisée en ce que** l'acide nucléique est un acide ribonucléique.

24. Composition selon l'une des revendications 22 ou 23 **caractérisée en ce que** l'acide nucléique est modifié chimiquement.

25. Composition selon la revendication 1 **caractérisée en ce que** l'acide nucléique est un antisens.

26. Composition selon l'une des revendications précédentes **caractérisée en ce que** l'acide nucléique comporte un gène thérapeutique.

27. Composition selon l'une des revendications précédentes **caractérisée en ce qu'**elle comprend en outre un adjuvant de type dioleoylphosphatidyléthanolamine (DOPE), l'oléoyl-palmitoylphos-phatidyléthanolamine (POPE), les di-stéaroyl, -palmitoyl,-mirystoyl phosphatidyléthanolamines ainsi que leurs dérivé N-méthylés 1 à 3 fois; les phosphatidylglycérols, les diacylglycérols, les glycosyldiacylglycérols, les cérébrosides (tels que notamment les galactocérébrosides), les sphingolipides (tels que notamment les sphingomyélines) ou encore les asialogangliosides.

28. Composition selon l'une des revendications précédentes **caractérisée en ce qu'**elle associe en outre à l'agent de transfection cationique un élément de ciblage.

29. Composition selon la revendication 28 **caractérisée en ce que** cet élément de ciblage est choisi parmi les anticorps dirigés contre des molécules de la surface cellulaire, des ligands de recepteurs membranaires comme l'insuline, la transferrine, l'acide folique ou tout autre facteur de croissance, cytokines ou vitamines. des lectines, modifiées ou non, des protéines à motif RGD, des peptides contenant un tandem de motifs RGD, cyclique ou non, des peptides polylysine ainsi que des peptides ligands naturels ou synthétiques.

30. Procédé de préparation d'une composition comprenant des particules de complexes agent(s) de transfection cationique (s)/acides nucléiques telle que définie précédemment **caractérisé en ce que** l'agent transfectant et l'acide nucléique sont mis en contact, en présence d'une quantité suffisante d'un agent de surface non ionique pour stabiliser les particules de complexes nucléiques ainsi formées à une taille inférieure à environ 160 nm.

31. Procédé selon la revendication 30 **caractérisé en ce que** l'un des composants choisi parmi l'acide nucléique ou le lipofectant est au préalable mélangé à l'agent de surface non ionique avant d'être mis en présence avec le second composant.

## Claims

1. Composition **characterized in that** it comprises particles of cationic transfection agent(s)/nucleic acid lamellar complexes, and **in that** it comprises at least one nonionic surface-active agent in a quantity of between 0.01% and 10% weight/volume, so as to obtain stabilized lamellar complexes with a diameter of less than or equal to 160 nm.

2. Composition according to Claim 1, **characterized in that** the surface-active agent comprises at least one hydrophobic segment and at least one hydrophilic segment.

3. Composition according to Claim 2, **characterized in that** the hydrophobic segment is chosen from aliphatic chains, polyoxyalkylenes, alkylidene polyesters, polyethylene glycols with a benzyl polyether head, and cholesterol.

4. Composition according to Claim 2, **characterized in that** the hydrophilic segment is chosen from polyoxyalkylenes, polyvinyl alcohols, polyvinyl-pyrrolidones, or saccharides.

5. Composition according to one of Claims 1 to 4, **characterized in that** the surface-active agent is a polyoxyalkylene of general formula:
HO(CH₂CH₂O)ₐ(CH(CH₃)CH₂O)_{b}(CH₂CH₂O)_{c}H
with a, b and c representing, independently of each other, integers which may vary between 20 and 100.

6. Composition according to Claim 5, **characterized in that** it contains, as surface-active agent, a compound of general formula OH(CH₂CH₂O)ₐ(CH(CH₃)CH₂O)_{b}(CH₂CH₂O)_{c}H, with a equal to 75, b to 30 and c to 75.

7. Composition according to one of Claims 1 to 4, **characterized in that** it contains, as surface-active agent, a compound of the family of polyethylene glycols with a dendritic benzyl polyether head.

8. Composition according to one of Claims 1 to 4, **characterized in that** it contains, as surface-active agent, a compound of the polyoxyethylene alcohol family.

9. Composition according to one of Claims 1 to 4, **characterized in that** it contains, as surface-active agent, polyoxyethylene nonylphenyl ether.

10. Composition according to Claim 1, **characterized in that** the surface-active agent is present in a quantity of between 0.02% and 5% weight/volume.

11. Composition according to Claim 1, **characterized in that** the cationic transfection agent is a lipofectant.

12. Composition according to Claim 11, **characterized in that** the lipofectant is an amphiphilic molecule comprising at least one lipophilic region combined or otherwise with a hydrophilic region.

13. Composition according to either of Claims 11 and 12, **characterized in that** it is a lipofectant comprising at least one polyamine region of general formula:
H₂N-(-(CH)ₘ-NH-)ₙ-H
in which m is an integer greater than or equal to 2 and n is an integer greater than or equal to 1, it being possible for m to vary between the different groups of carbon between 2 amines, this polyamine region being covalently combined with a lipophilic region of the saturated or unsaturated hydrocarbon chain type, cholesterol, or a natural or synthetic lipid capable of forming lamellar or hexagonal phases.

14. Composition according to Claim 13, **characterized in that** the polyamine region is represented by spermine or one of its analogues which has conserved its nucleic acid-binding properties.

15. Composition according to either of Claims 11 and 12, **characterized in that** it involves a lipofectant of general formula: 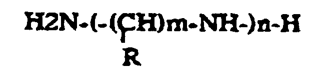 in which R denoting the lipophilic region is represented by the general formula: 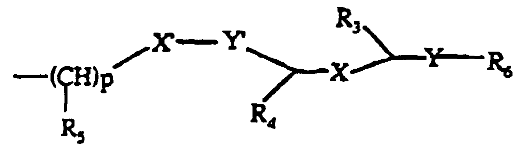 in which X and X' represent, independently of each other, an oxygen atom, a methylene group -(CH₂)_{q}-, with q equal to 0, 1, 2 or 3, or an amino group -NH- or -NR'-, with R' representing a C₁ to C₄ alkyl group, Y and Y' represent, independently of each other, a methylene group, a carbonyl group or a group C=S, R₃, R₄ and R₅ represent, independently of each other, a hydrogen atom or a substituted or unsubstituted C₁ to C₄ alkyl radical, with p capable of varying between 0 and 5, R₆ represents a cholesterol derivative or an alkylamino group -NR₁R₂, with R₁ and R₂ representing, independently of each other, a saturated or unsaturated, linear or branched C₁₂ to C₂₂ aliphatic radical.

16. Composition according to either of Claims 11 and 12, **characterized in that** it involves a lipofectant of general formula: 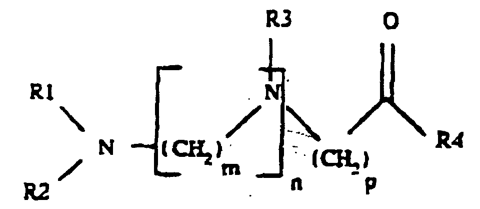 in which R₁, R₂ and R₃ represent, independently of each other, a hydrogen atom or a group -(CH₂)_{q}-NRR', with q being capable of varying between 1, 2, 3, 4, 5 and 6, independently between the different groups R₁, R₂ and R₃ and R and R' representing, independently of each other, a hydrogen atom or a group -(CH₂)_{q'}-NH₂, q' being capable of varying between 1, 2, 3, 4, 5 and 6, independently between the different groups R and R', m, n and p represent, independently of each other, an integer capable of varying between 0 and 6 with, when n is greater than 1, m being capable of taking different values and R₃ different meanings within the preceding general formula and R₄ represents a group of general formula: 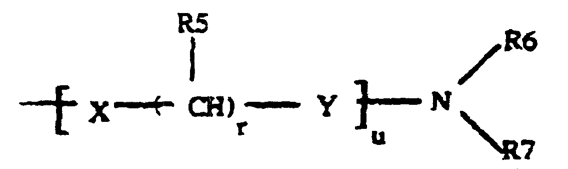 in which R₆ and R₇ represent, independently of each other, a hydrogen atom or a saturated or unsaturated C₁₀ to C₂₂ aliphatic radical with at least one of the two groups being different from hydrogen, u is an integer chosen between 0 and 10 with, when u is an integer greater than 1, R₅, X, Y and r being capable of having different meanings within the different units [X-(CHR₅)ᵣ-Y], X represents an oxygen or sulphur atom, or an amine group which is monoalkylated or otherwise, Y represents a carbonyl group or a methylene group, R₅ represents a hydrogen atom or a natural amino acid side chain, substituted if appropriate, and r represents an integer varying between 1 and 10 with, when r is equal to 1, R₅ representing a substituted or unsubstituted natural amino acid side chain, and when r is greater than 1, R₅ representing a hydrogen atom.

17. Composition according to either of Claims 11 and 12, **characterized in that** the said lipofectant is a cationic lipid carrying one or more guanidinium and/or amidinium groups.

18. Composition according to one of Claims 1 to 10, **characterized in that** the cationic transfection agent is a cationic polymer.

19. Composition according to Claim 18, **characterized in that** the said cationic polymer is a compound of general formula (I): 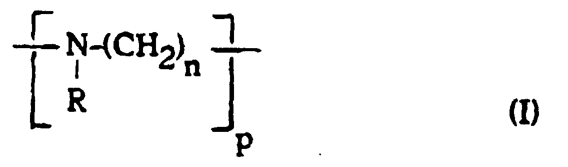 in which R may be a hydrogen atom or a group of formula: 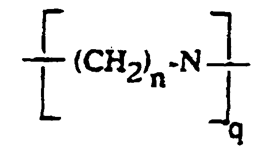 n being an integer between 2 and 10, p and q being integers, it being understood that the sum p+q is such that the average molecular weight of the polymer is between 100 and 10⁷ Da.

20. Composition according to either of Claims 18 and 19, **characterized in that** it involves the polyethylene imine of average molecular weight 50,000 Da (PEI50K), the polyethylene imine of average molecular weight 22,000 Da (PEI22K) or the polyethylene imine of average molecular weight 800,000 Da (PEI800K).

21. Composition according to one of Claims 1 to 11, **characterized in that** the cationic transfection agent is preferably chosen from lipofectamine, dioctadecylamidoglycyl spermine (DOGS), palmitoylphosphatidylethanolamine 5-carboxyspermylamide (DPPES), 2,5-bis(3-aminopropylamino)pentyl (dioctadecylcarbamoylmethoxy)-acetate or 1,3-bis(3-aminopropylamino)-2-propyl (dioctadecylcarbamoylmethoxy)acetate, {H₂N(CH₂)₃}₂N-(CH₂)₄N{(CH₂)₃NH₂}(CH₂)₃NHCH₂COGlyN[(CH₂)₁₇CH₃]₂, H₂N(CH₂)₃NH(CR₂)₄NH(CH₂)₃NHCH₂COGlyN[(CH₂)₁₇CH₃]₂, and H₂N(CH₂)₃NH(CH₂)₄NH(CH₂)₃NHCH₂COArgN [(CH₂)₁₇CH₃]₂.

22. Composition according to Claim 1, **characterized in that** the nucleic acid is a deoxyribonucleic acid.

23. Composition according to Claim 1, **characterized in that** the nucleic acid is a ribonucleic acid.

24. Composition according to either of Claims 22 and 23, **characterized in that** the nucleic acid is chemically modified.

25. Composition according to Claim 1, **characterized in that** the nucleic acid is an antisense nucleic acid.

26. Composition according to one of the preceding claims, **characterized in that** the nucleic acid comprises a therapeutic gene.

27. Composition according to one of the preceding claims, **characterized in that** it comprises, in addition, an adjuvant of the type comprising dioleoylphosphatidylethanolamine (DOPE), oleoylpalmitoylphosphatidylethanolamine (POPE), di-stearoyl, -palmitoyl, -myristoyl phosphatidylethanolamines as well as their derivatives which are N-methylated 1 to 3 times, phosphatidylglycerols, diacylglycerols, glycosyldiacylglycerols, cerebrosides (such as in particular galactocerebrosides), sphingolipids (such as in particular sphingomyelins) or alternatively asialogangliosides.

28. Composition according to one of the preceding claims, **characterized in that** it combines, in addition, a targeting element with the' cationic transfection agent.

29. Composition according to Claim 28, **characterized in that** this targeting element is chosen from antibodies directed against molecules of the cellular surface, membrane receptor ligands such as insulin, transferrin, folic acid or any other growth factor, cytokines or vitamins, lectins, modified or otherwise, proteins with an RGD unit, peptides containing a tandem array of RGD units, cyclic or otherwise, polylysine peptides as well as natural or synthetic ligand peptides.

30. Process for the preparation of a composition comprising particles of cationic transfection agent(s)/nucleic acid complexes as defined hereinabove, **characterized in that** the transfecting agent and the nucleic acid are brought into contact in the presence of a sufficient quantity of a nonionic surface-active agent to stabilize the particles of nucleic complexes thus formed at a size of less than about 160 nm.

31. Process according to Claim 30, **characterized in that** one of the components chosen from the nucleic acid or the lipofectant is mixed beforehand with the nonionic surface-active agent before being brought into contact with the second component.

## Patentansprüche

1. Zusammensetzung, **dadurch gekennzeichnet, dass** sie Teilchen aus lamellären Komplexen kationische(s) Transfektionsmittel/Nukleinsäuren umfasst und dass sie mindestens ein nichtionisches oberflächenaktives Mittel in einer Menge zwischen 0,01 und 10 Gew.-%/Volumen umfasst, so dass stabilisierte lamelläre Komplexe mit einem Durchmesser von kleiner oder gleich 160 nm erhalten werden.

2. Zusammensetzung nach Anspruch 1, **dadurch gekennzeichnet, dass** das oberflächenaktive Mittel mindestens ein hydrophobes Segment und mindestens ein hydrophiles Segment umfasst.

3. Zusammensetzung nach Anspruch 2, **dadurch gekennzeichnet, dass** das hydrophobe Segment aus aliphatischen Ketten, Polyoxyalkylenen, Alkylidenpolyestern, Polyethylenglykol mit Benzylpolyetherkopf und Cholesterin ausgewählt ist.

4. Zusammensetzung nach Anspruch 2, **dadurch gekennzeichnet, dass** das hydrophile Segment aus Polyoxyalkylenen, Polyvinylalkoholen, Polyvinylpyrrolidonen oder Sacchariden ausgewählt ist.

5. Zusammensetzung nach einem der Ansprüche 1 bis 4, **dadurch gekennzeichnet, dass** das oberflächenaktive Mittel ein Polyoxyalkylen der allgemeinen Formel:
HO(CH₂CH₂O)ₐ(CH(CH₃)CH₂O)_{b}(CH₂CH₂O)_{c}H
ist, wobei a, b und c unabhängig voneinander ganze Zahlen, die zwischen 20 und 100 variieren können, bezeichnen.

6. Zusammensetzung nach Anspruch 5, **dadurch gekennzeichnet, dass** sie als oberflächenaktives Mittel eine Verbindung der allgemeinen Formel OH(CH₂CH₂O)ₐ(CH(CH₃)CH₂O)_{b}(CH₂CH₂O)_{c}H umfasst, wobei a gleich 75, b gleich 30 und c gleich 75 ist.

7. Zusammensetzung nach einem der Ansprüche 1 bis 4, **dadurch gekennzeichnet, dass** sie als oberflächenaktives Mittel eine Verbindung der Polyethylenglykol-Familie mit einem dendritischen Benzylpolyetherkopf umfasst.

8. Zusammensetzung nach einem der Ansprüche 1 bis 4, **dadurch gekennzeichnet, dass** sie als oberflächenaktives Mittel eine Verbindung der Polyoxyethylenalkohol-Familie enthält.

9. Zusammensetzung nach einem der Ansprüche 1 bis 4, **dadurch gekennzeichnet, dass** sie als oberflächenaktives Mittel Polyoxyethylennonylphenylether enthält.

10. Zusammensetzung nach Anspruch 1, **dadurch gekennzeichnet, dass** das oberflächenaktive Mittel in einer Menge zwischen 0,02 und 5 Gew.-%/Volumen vorhanden ist.

11. Zusammensetzung nach Anspruch 1, **dadurch gekennzeichnet, dass** das kationische Transfektionsmittel ein Lipofektant ist.

12. Zusammensetzung nach Anspruch 11, **dadurch gekennzeichnet, dass** das Lipofektant ein amphiphiles Molekül, umfassend mindestens eine lipophile Region, die mit einer hydrophilen Region assoziiert ist oder nicht assoziiert ist, ist.

13. Zusammensetzung nach einem der Ansprüche 11 oder 12, **dadurch gekennzeichnet, dass** es sich um ein Lipofektant handelt, das mindestens eine Polyaminregion der allgemeinen Formel:
H₂N-(-(CH)ₘ-NH-)ₙ-H
umfasst, worin m eine ganze Zahl von größer oder gleich 2 ist und n eine ganze Zahl von größer oder gleich 1 ist, wobei m zwischen den verschiedenen Kohlenstoffgruppen, die zwischen 2 Aminen in covalenter Assoziierung an eine lipophile Region vom Typ Kohlenwasserstoff, der gesättigt oder nicht-gesättigt sein kann, Cholesterin oder ein natürliches oder synthetisches Lipid, das lamelläre oder hexagonale Phasen bilden kann, vorhanden sind, variieren kann.

14. Zusammensetzung nach Anspruch 13, **dadurch gekennzeichnet, dass** die Polyaminregion durch Spermin oder eines seiner Analoga, das seine Eigenschaften zur Bindung an die Nukleinsäure beibehalten hat, wiedergegeben ist.

15. Zusammensetzung nach einem der Ansprüche 11 oder 12, **dadurch gekennzeichnet, dass** es sich um ein Lipofektant der allgemeinen Formel: 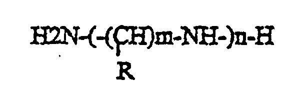 handelt, worin R, das die lipophile Region darstellt, durch die allgemeine Formel: 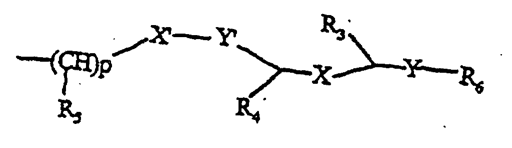 wiedergegeben wird, worin X und X' unabhängig voneinander ein Sauerstoffatom, eine Methylengruppe -(CH₂)_{q}-, wobei q 0, 1, 2 oder 3 bedeutet, oder eine Aminogruppe -NH- oder-NR'-, wobei R' eine C₁-C₄-Alkylgruppe bedeutet, darstellen, Y und Y' unabhängig voneinander eine Methylengruppe, eine Carbonylgruppe oder eine Gruppierung C=S bedeuten, R₃, R₄ und R₅ unabhängig voneinander ein Wasserstoffatom oder einen C₁-C₄-Alkylrest, der substituiert sein kann oder nicht, wobei p zwischen 0 und 5 variieren kann, bedeuten, R₆ ein Cholesterinderivat oder eine Alkylaminogruppe -NR₁R₂ bedeutet, wobei R₁ und R₂ unabhängig voneinander einen gesättigten oder ungesättigten, linearen oder verzweigten aliphatischen C₁₂-C₂₂-Rest bezeichnen.

16. Zusammensetzung nach einem der Ansprüche 11 oder 12, **dadurch gekennzeichnet, dass** es sich um ein Lipofektant der allgemeinen Formel: 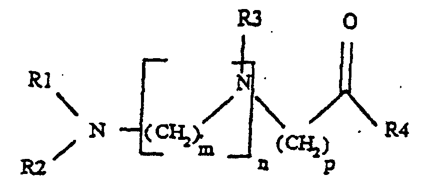 handelt, worin R₁, R₂ und R₃ unabhängig voneinander ein Wasserstoffatom oder eine Gruppierung -(CH₂)_{q}-NRR' bedeuten, wobei q zwischen 1, 2, 3, 4, 5 und 6 und unabhängig zwischen den verschiedenen Gruppierungen R₁, R₂ und R₃ variieren kann, und R und R' unabhängig voneinander ein Wasserstoffatom oder eine Gruppierung -(CH₂)_{q}'-NH₂ bedeuten, q' zwischen 1, 2, 3, 4, 5 und 6 und unabhängig zwischen den verschiedenen Gruppierungen R und R' variieren kann, m, n und p unabhängig voneinander eine ganze Zahl bezeichnen, die zwischen 0 und 6 variieren kann, wobei, wenn n einen Wert über 1 hat, m verschiedene Werte annehmen kann und R₃ verschiedene Bedeutungen in der vorstehenden allgemeinen Formel besitzen kann, und R₄ eine Gruppierung der allgemeinen Formel: 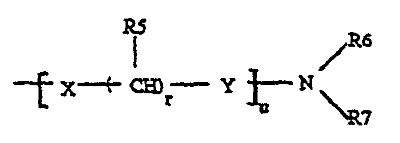 bedeutet, worin R₆ und R₇ unabhängig voneinander ein Wasserstoffatom oder einen aliphatischen, gesättigten oder nicht-gesättigten C₁₀-C₂₂-Rest bedeuten, wobei mindestens eine der zwei Gruppierungen von Wasserstoff verschieden ist, u eine ganze Zahl, ausgewählt zwischen 0 und 10 ist, wobei, wenn u eine ganze Zahl über 1 bedeutet, R₅, X, Y und r verschiedene Bedeutungen in den verschiedenen Motiven [X-(CHR₅)ᵣ-Y] besitzen können, X ein Sauerstoff- oder Schwefelatom oder eine monoalkylierte oder nicht-monoalkylierte Amingruppe bedeutet, Y eine Carbonylgruppierung oder eine Methylengruppierung bedeutet, R₅ ein Wasserstoffatom oder eine Seitenkette einer natürlichen Aminosäure bedeutet, die gegebenenfalls substituiert ist, und r eine ganze Zahl, die zwischen 1 und 10 variieren kann, bedeutet, wobei, wenn r einen Wert von 1 hat, R₅ eine Seitenkette einer natürlichen Aminosäure, die substituiert sein kann oder nicht, bedeutet, und wenn R einen Wert über 1 hat, R₅ ein Wasserstoffatom bedeutet.

17. Zusammensetzung nach einem der Ansprüche 11 oder 12, **dadurch gekennzeichnet, dass** das Lipofektant ein kationisches Lipid ist, das ein oder mehrere Guanidinium- und/oder Amidiniumgruppierungen trägt.

18. Zusammensetzung nach einem der Ansprüche 1 bis 10, **dadurch gekennzeichnet, dass** das kationische Transfektionsmittel ein kationisches Polymeres ist.

19. Zusammensetzung nach Anspruch 18, **dadurch gekennzeichnet, dass** das kationische Polymere eine Verbindung der allgemeinen Formel (I): 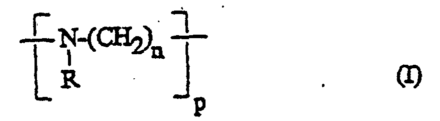 ist, worin R ein Wasserstoffatom oder eine Gruppe der Formel: 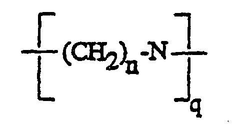 sein kann, n eine ganze Zahl zwischen 2 und 10 ist und p und q ganze Zahlen sind, mit der Maßgabe, dass die Summe p+q so ist, dass das mittlere Molekulargewicht der Polymeren zwischen 100 und 10⁷ Da liegt.

20. Zusammensetzung nach einem der Ansprüche 18 oder 19, **dadurch gekennzeichnet, dass** es sich um ein Polyethylenimin mit einem mittleren Molekulargewicht von 50.000 Da (PEI50K), Polyethylenimin mit einem mittleren Molekulargewicht von 22.000 Da (PEI22K) oder Polyethylenimin mit einem mittleren Molekulargewicht von 800.000 Da (PEI800K) handelt.

21. Zusammensetzung nach einem der Ansprüche 1 bis 11, **dadurch gekennzeichnet, dass** das kationische Transfektionsmittel bevorzugt aus Lipofectamin, Dioctadecylamidoglycylspermin (DOGS), Palmitoylphosphatidylethanolamin-5-carboxyspermylamid (DPPES), 2,5-Bis-(3-aminopropylamino)pentyl(dioctadecylcarbamoylmethoxy)acetat oder 1,3-Bis-2-(3-aminopropylamido)propyl(dioctadecylcarbamoylmethoxy)acetat, {H₂N(CH₂)₃}₂N(CH₂)₄N{(CH₂)₃NH₂}(CH₂)₃NHCH₂COGlyN[(CH₂)₁₇CH₃]₂, H₂N(CH₂)₃NH(CH₂)₄NH(CH₂)₃NHCH₂COGlyN[(CH₂)₁₇CH₃]₂ und H₂N(CH₂)₃NH(CH₂)₄NH(CH₂)₃NHCH₂COArgN[(CH₂)₁₇CH₃]₂ ausgewählt ist.

22. Zusammensetzung nach Anspruch 1, **dadurch gekennzeichnet, dass** die Nukleinsäure eine Desoxyribonukleinsäure ist.

23. Zusammensetzung nach Anspruch 1, **dadurch gekennzeichnet, dass** die Nukleinsäure eine Ribonukleinsäure ist.

24. Zusammensetzung nach einem der Ansprüche 22 oder 23, **dadurch gekennzeichnet, dass** die Nukleinsäure chemisch modifiziert ist.

25. Zusammensetzung nach Anspruch 1, **dadurch gekennzeichnet, dass** die Nukleinsäure eine Antisense ist.

26. Zusammensetzung nach einem der vorstehenden Ansprüche, **dadurch gekennzeichnet, dass** die Nukleinsäure ein therapeutisches Gen umfasst.

27. Zusammensetzung nach einem der vorstehenden Ansprüche, **dadurch gekennzeichnet, dass** sie weiterhin ein Adjuvans vom Typ Dioleoylphosphatidylethanolamin (DOPE), Oleoylpalmitoylphosphatidylethanolamin (PO-PE), Distearoyl, -palmitoyl, -myristoylphosphatidylethanolamin sowie ihre 1- bis 3-fach N-methylierten Derivate, Phosphatidylglycerine, Diacylglycerine, Glykosyldiacylglycerine, Cerebroside (wie insbesondere Galactocerebroside), Sphingolipide (wie insbesondere Sphingomyeline) oder auch Asialoganglioside umfasst.

28. Zusammensetzung nach einem der vorstehenden Ansprüche, **dadurch gekennzeichnet, dass** sie in weiterer Assoziation mit dem kationischen Transfektionsmittel ein Element zum Durchmustern umfasst.

29. Zusammensetzung nach Anspruch 28, **dadurch gekennzeichnet, dass** das Element zum Durchmustern ausgewählt ist aus gegen Moleküle der cellulären Oberfläche gerichteten Antikörpern, Liganden von Membranrezeptoren, wie Insulin, Transferrin, Folsäure oder jeder andere Wachstumsfaktor, Cytokine oder Vitamine, modifizierten oder nicht-modifizierten Lectinen, Proteinen mit einem RGD-Motiv, Peptiden, die ein Tandem von RGD-Motiven enthalten und cyclisch oder nicht-cyclisch sind, Polylysinpeptiden sowie natürlichen oder synthetischen Peptidliganden.

30. Verfahren zur Herstellung einer Zusammensetzung, umfassend Teilchen der Komplexe kationische(s) Transfektionsmittel/Nukleinsäuren, wie vorstehend definiert, **dadurch gekennzeichnet, dass** das Transfektionsmittel und die Nukleinsäure in Gegenwart einer ausreichenden Menge eines nichtionischen oberflächenaktiven Mittels in Kontakt gebracht werden, um die so gebildeten Teilchen mit einer Größe von kleiner etwa 160 nm aus Nukleinsäurekomplexen zu stabilisieren.

31. Verfahren nach Anspruch 30, **dadurch gekennzeichnet, dass** einer der Bestandteile, ausgewählt aus Nukleinsäure oder Lipofektant, zuvor mit dem nichtionischen oberflächenaktiven Mittel vermischt wird, bevor er in Gegenwart des zweiten Bestandteils gebracht wird.
